Europäisches Patentamt

European Patent Office

Office européen des brevets

(11) Publication number: **0 278 473 B1**

(12) **EUROPEAN PATENT SPECIFICATION**

(45) Date of publication of patent specification: **05.08.92**   (51) Int. Cl.⁵: **A61K 37/02, A61N 1/30**

(21) Application number: **88101856.8**

(22) Date of filing: **09.02.88**

(54) **Electrolytic transdermal delivery of proteins.**

(30) Priority: **10.02.87 US 12898**

(43) Date of publication of application:
**17.08.88 Bulletin 88/33**

(45) Publication of the grant of the patent:
**05.08.92 Bulletin 92/32**

(84) Designated Contracting States:
**AT BE CH DE ES FR GB IT LI LU NL SE**

(56) References cited:
**EP-A- 0 092 015**
**WO-A-86/02277**
**CH-A- 631 625**
**US-A- 4 474 570**
**US-A- 4 640 689**

**Journal of pharmaceutical sciences, vol. 72, no. 3, march 1983, Washington S. SATO et al. "Prevention of Insulin Self-Association and Surface Adsorption"**

(73) Proprietor: **Drug Delivery Systems Inc.**
**292 Madison Avenue**
**New York New York 10017(US)**

(72) Inventor: **Sibalis, Dan**
**268 Hallock Road**
**Stony Brook New York 11790(US)**
Inventor: **Rosen, Sanford**
**64 E. 86th Street**
**New York New York 10028(US)**

(74) Representative: **Patentanwälte RUFF, BEIER und SCHÖNDORF**
**Neckarstrasse 50**
**W-7000 Stuttgart 1(DE)**

Rank Xerox (UK) Business Services

**Description**

This invention relates to electrolytic transdermal delivery of proteins and more specifically to delivery to the blood stream of the patient of aqueous solutions or suspensions of insulin in the presence of an agent with negative Setschenow constants.

A number of patents disclose transdermal electrical, and medical effects, as follows:

| U.S. Patents | | |
| --- | --- | --- |
| 385,556 | 2,267,162 | 3,163,166 |
| 486,902 | 2,493,155 | 3,289,671 |
| 588,479 | 2,784,715 | 3,547,107 |
| 3,677,268 | 4,239,052 | 4,367,745 |
| 4,008,721 | 4,243,052 | 4,367,745 |
| 4,141,358 | 4,273,135 | 4,406,658 |
| 4,164,226 | 4,290,878 | 4,419,019 |
| 4,166,457 | 4,325,367 | 4,474,570 |
| 4,239,046 | 4,362,645 | 4,557,723 |
| 4,622,031 | 4,640,689 | |
| EPA 58,920 | DE 2,902,021.83 | UK 2,104,388 |
| EPA 60,452 | DE 3,225,748 | |

None of these references, however, show the effective administration of protein drugs; especially high molecular weight proteins such as insulin, renin, corticortropin, calcitonin, and glucogon with structures having more than about 20 polypeptide units and molecular weights up to 40,000 daltons.

A paper entitled "Prevention of Insulin Self-Association and Surface Adsorption" by Sato, Ebert, and Kim in the Journal of Pharmaceutical Sciences 72, No. 3, 228-232, March 1983 discusses the association of insulin in water to dimers, tetramers, hexamers, and higher aggregates and consequent adsorption of these polymers onto plastic surfaces such as polyurethane, silicone, rubber, and cellulose under high shear and in the presence of additives such as lysine, ethylenediaminetetraacetic acid salts, and urea.

The ionophoresis of conventional commercial insulin is termed "meaningless" with "no significant differences in changing blood glucose levels" in a paper by Stephen, Peterlenz, and Jacobsen in Biomedical Biochemical Acta 5, 553-558 (1984) experimenting on eight human volunteers. One pig was treated with an unspecified, modified, highly ionized, predominately monomeric, derivative of insulin for 20 minutes followed by a drop in blood sugar. This publication states that "a new ionized form of insulin must be synthesized" in order for ionophoresis to be possible and states the permeability of human skin to high molecular weight polymers to be "questionable". Other difficulties discussed in this paper are the association of commercial insulin rendering the impermeability to insulin of human skin as "almost certain" and the weak ionization of insulin as "mitigating against successful transcutaneous electronic (sic) transfer of the drug".

Objects of the Invention

It is an object of the present invention to use normal, unmodified protein drugs for the manufacture of compositions administered transdermally to humans and other animal patients by means of a locally applied electric field.

It is a further object of the invention to permit the administration of protein drugs transdermally in an electric field regardless of the degree of ionization or the amount of ionic charge on the protein.

It is yet another object to thereby maximize the transdermal administration of protein drugs by eliminating or minimizing the association of protein drugs in aqueous media

It is still another object to permit the administration of protein drugs transdermally by an electric applicator which occupies minimal area, gives the patient minimal discomfort, generates sufficient current density with minimal size and weight, and operates effectively under a wide variety of skin conditions.

It is yet a further object to permit the administration of protein drugs transdermally by electrolytic devices without irritation or reddening of the skin, and without tingling or other sensations.

Other objects of the present invention will be apparent to those skilled in the art.

The present invention utilizes a chemical composition for transdermal transport to the blood stream of the patient by electrolytic means comprising:

2

a) a protein,

b) a cosolvent with negative Seschenow constants, and

c) water.

Protein comprise natural and synthetic modifications of different solubility: albumins, globulins, prolamines and of different functional forms: enzymes, hemoglobin, hormones, viruses, genes, antibodies, and nucleic acids, and polypeptides with more than 20 aminoacid units.

Proteins vary in molecular weight from polypeptides of about 2000 daltons to a single strand of insulin, about 5800 daltons, to megamolecules such as tobacco mosaic virus, about 40 million daltons.

The agent for dissociating protein aggregates preferably has negative interaction parameters for both the average peptide and average methylene group (Setschenow constants), their sum, and also a negative standard free energy of transfer from water to a solution of that agent.

Water means liquid water at normal, ambient temperature, pressure, and other possible conditions (e.g. gravity) with no perturbations (e.g. no radiation, electric, magnetic, or any other possible fields).

Other useful additives may also be present, such as buffers, biocides, preservatives, or stabilizers.

The chemical composition of the present invention is intended for use in a transdermal electrolytic applicator comprising:

at least two electrode elements forming the applicator and separated by barrier means,

reservoir means containing the chemical composition of this invention,

an electronic/electrolytic circuit, including a power source, for supplying electric power to the electrodes and reservoir,

cover means partially enclosing at least the reservoir means, and

adhesive means for holding the applicator to the patient's skin, whereby the protein may be transported from the reservoir through the skin to the patient's bloodstream.

Fig. 1 is a cross-sectional view of one type of electronic/electrolytic drug applicator employing the chemical composition of the present invention to administer proteins to a patient transdermally.

Fig. 2 and 3 show blood glucose and serum insulin levels in diabetic rabbits.

## DESCRIPTION OF THE PREFERRED EMBODIMENTS OF THE INVENTION

Simple proteins are homo or hetero condensation polymers or aminoacids which are linked by formation of amide bonds from the amino group of one aminoacid and the carboxylic acid group of another. Conjugate proteins contain amino acid moieties plus nucleic acids, carbohydrates, lipids, or other chemical classes. Proteins nay be fibrous, globular, pigmented by metals, crosslinked, or aggregative. The 20 alphaamino acids necessary for mammalian life constitute essentially all the "building blocks" of protein macromolecules.

Proteins can vary widely in molecular weight from a few thousand daltons, as in glucagon (29 peptide units), through tens of thousands of daltons, as in six-stranded insulin, to millions of daltons as in viruses. In order for large proteins to be transported through the skin, it is preferable that the protein be unassociated. If the protein is single-stranded, the cross-section of the linear macromolecular would be only a few square Angstoms even if the entire folded, convoluted, or random polymer is large in radius of gyration.

Insulin is a highly studied protein illustrating the importance of nonassociation. One multi-looped strand of insulin is a polypeptide with 51 aminoacid units having a molecular weight of about 5800 daltons. Normal commercially available insulin, whether human, bovine, or porcine, is an association of six such multi-looped strands having a molecular weight of about 35,000 daltons. In order for insulin to be delivered transdermally in controlled meaningful amounts, it is preferable that it be totally unassociated in an electrolytic transdermal device. Until this invention, a composition and device for solving this problem had not be found. Other proteins which nay be transdermally delivered to the patient are glucagon, calcitonin, protamine, adrenal cortex hormones and various globulin fractions, and the like.

In order to appreciate the importance of the present invention resulting in the nonassociation of proteins in water, it is helpful to understand the structure of proteins and of liquid water. Water is not a simple liquid, as is for example liquid argon. The HOH molecule is an isosceles triangle with a central angle of 104.52°; the H-O distance is about 0,141 nm. Liquid water has a tetrahedral structure with an O-O distance of 0,276 nm, an angle of 109.46°, and highly hybridized 2s and 2p orbitals. There are in fact nine different forms of ice structures, illustrating the variety of structures possible for water liquid. Since ice contracts on melting between 0 and 4°C, water has an "open" structure (cf. germanium, which also contracts on melting). Water has strong, directional forces which lead to a high dielectric constant for its nonideal structure. Ammonia and methane which have molecular weights similar to water are gases at ambient conditions not liquid, thus showing the difference between normal liquids and the strong dipole-dipole interaction in liquid water which

EP 0 278 473 B1

has a high boiling point, high melting point, high enthalpy and high entropy of vaporization.

There is a strong repulsion between water molecules at an "interpenetration" distance of less than 0,2 nm. At intermediate distance of 0,2 to 0,5 nm there is strong hydrogen bonding between water molecules, which have a coordination number of 4. The enthalpy of sublimation of ice is 11.65 kcal/mole, much higher than simple dipole interactions, but much less than that of a chemical bond.

There have been many proposed descriptive models for the structure of liquid water, such as:

a) in interstitial model of ice whose cavities are filled with water,

b) quartz-like aggregates,

c) water as a hydrate of itself,

d) flickering clusters of cooperative H-bonds;

e) a two-structure, mixture model.

Chemists have known for decades that water forms clathrates with xenon, chlorine, methane and other molecules and therefore must have cavities. The structure of liquid water depends on the distance and angles of H-bonds. In a two-dimensional sense, water is a hexagonal array of "aromatic" structures.

The solubility of argon in "structured" water is about one-tenth that of argon in alcohols. As temperature increases from 0 to 30°C. the solubility of argon in water decreases while that in alcohols increases, thus showing a decrease in water's cavities from 0 to 30°C. The change in entropy of methane in water is about -15 to -20 e.u. but in alcohols, dioxane, and cyclohexane is about -1 e.u. The change in enthalpy of methane in water is about -3000 cal/mole, but in the same organics is 200-500 cal/mole. The process of solution may be modeled as "forming a cavity" then introducing the solute into that cavity. In a normal fluid the energy to form the cavity is positive, then filling the cavity is minus (attractive). Since water already has cavities present, there is about zero energy to form such and negative energy to dissolve the solute (fill) the cavity.

Adding some nonpolar nonelectrolytes such as ether, methyl acetate, dimethylsulfoxide structure water i.e. reinforce water structure and decrease its compressbility. Some small ions e.g. lithium and fluoride also reinforce the structure of water.

Conversely, most ions, iodine, methyl halides, small aminoacids, urea, and other polar nonelectrolytes are "structure breakers" of water.

Precise analysis of the structure of water may be a complex matter, yet it is a description of only one substance. Precise description of proteins dissociating if already dissolved, covers much broader phenomena, since there are many proteins and many cosolvents or dissociation agents to coact with water.

Just a litany of some of the terms used to name the phenomenon hints at the problems in precisely describing the interaction of protein/agent/water:

Association/disassociation, self-association, polymerization/depolymerization, gelation, intracellular aggregation, binding, multidomain folding, hydrophobic stabilization, helix promotion, random coil promotion, denaturation, lyophilizing, perturbation, demetalization, hydrophobic interactions, subunit contacts, assemblies, and molecular weight transitions.

The conformation of proteins in solution is dependent at a minimum on the concentration of protein, pH, solvent composition, ionic strength, ionic charge on the protein, solvent dielectric properties, presence of cosolutes, shear stresses, and the presence of heterogeneous third bodies such as surfaces of the container, granules, and the like.

It is generally accepted that the configuration of proteins in aqueous media comprises folded macromolecules with hydrophobic domains forming a central core and hydrophilic domains oriented toward the aqueous perimeter. The process of dissolution is difficult to describe in detail, but the energetics of the solution process can be determined in a straightforward manner. Much information about solution, disassociation, denaturation, coiling, gelation, unfolding, and other changes in so-called tertiary and quaternary structures may be gained from a detailed study of solution and/or gelation of proteins in water and water containing other cosolvents or "agents".

The primary structure of a protein is the term used for the sequence of aminoacids as they appear along the chain of the macromolecule. The local organization of the chain e.g. helix formation, random coil, folding is termed secondary structure. The overall spatial arrangement of the protein on the atomic level, what X-ray crystallography shows, is the tertiary level of structure. For insulin, this was published by Hodgkin et al in Nature, vol. 224, November 1, 1969 (Centenary issue) at pp. 491-495. The quaternary structure is that of several chains which may form different regions with different properties e.g. a dumbbell-like structure with a flexible middle rod and two hard ends. The function of the regions may vary. In hemoglobin, 4 myoglobins group to form a dumbbell shape with a molecular weight of about 17,000 daltons with the oxygen-bearing function associated with the two harder spheres on the ends rather than the flexible part in the middle.

4

The dissociation agents of the present invention greatly affect quaternary structure, are irrelevant to tertiary structure, may affect secondary structure, and have no effect on primary structure of polypeptides.

The effect of a solvent such as water on a protein can be described in terms of an equilibrium constant $K_d$ and the standard free energy of dissociation $F°$, when a protein dissociates from e g. hexamers to dimers or single-stranded subunits e.g. insulin in water. Often these different fragments can coexist in a series of equilibria e.g. earthworm hemoglobin can have duodecamers, hexamers, tetramers, diners, and single fragments, at the same time at intermediate concentrations of a pure solvent or one with a dissociation agent such as propylurea or sodium perchlorate present. When such an added dissociation cosolvent is present there are two dissociation constants $K_{DW}$ and $K_{DAW}$, where DW designates pure water and DAW designates added agent and water. Also, the interaction of the added agent and the protein involves the binding constant $K_B$.

For proteins binding constant $K_B$ is the summation of two terms: a polar component $K_P$ related to the peptide bond -NHCOO-and a hydrophobic component $K_H$ related to the average hydrophobic moiety -CHR-, different for each aminoacid but averagable. The constant is related to energetics by the Nernst equation. So

$$F°_{DW} = F°_{DAW} - mNRT (K_P + K_H)[da],$$

when m is the number of fragments and N is the number of binding sites and [da] is the concentration of the dissociation agent cosolvent.

When a solid protein is in contact With a well-stirred solvent such as water for a long time (e.g. a week) an equilibrium saturated solution is established:

$$K_{eq} = - RTlnC_{sat}$$

When another compound is added to the water, such as an electrolyte or a nonelectyrolyte, a different $C_{sat}$ is established at equilibrium. This other value will normally be different from $C_{sat}$ for pure water. The higher the concentration of the added agent, the higher (or lower) the saturated concentration of the protein. When one graphs the log $C_{sat}$ against the molarity of the added agent, a straight line is formed. The slope of this straight line is known as the Setschenow constant for that agent. Since the equation above has a minus sign in it, those agents which aid solubility and dissociation, e.g. urea or sodium perchlorate, have a negative Setschenow constant, and those agents which decrease solubility and dissociation, e.g. sodium or ammonium sulfate have positive Setschenow constants.

$$K_s \approx -K_B /2.303$$

The Setschenow constant $K_s$ has peptide and hydrophobic components. As shown above, the Setschenow constant can be approximated by negative $K_B$ divided by log transform constant 2.303. Since negative standard free energies of transfer indicate spontaneous reactions, negative $F°$ values for transfer from water to a mixture of water and the cosolvent indicate dissociation. The more negative, the more dissociated. Table I gives Setschenow constants for average peptide and hydrophobic groups, their sum, as well as free energy of transfer values for a variety of cosolvents, as taken from a paper by Herkovits et al. , Journal of Colloid and Interface Science, vol. 63, Ho. 2, p. 232 of February 1978. The lower the position in Table I, the better the dissociation agent.

EP 0 278 473 B1

Table I

| Setschenow Constants | | | | |
|---|---|---|---|---|
| Agent | For peptide | For -CH2- | Sum | -F° t cal/mo |
| Sodium sulfate | -0.013 | 0.085 | 0.072 | 98 |
| Potassium fluoride | -0.027 | 0.05 | 0.023 | 31 |
| Ethanol | +0.037 | -0.014 | 0.023 | 31 |
| Dioxane | +0.029 | -0.013 | 0.016 | 22 |
| Sodium chloride | -0.037 | 0.033 | -0.004 | -5 |
| Sodium acetate | - | - | -0.009 | -12 |
| Sodium bromide | -0.037 | 0.025 | -0.012 | -16 |
| Calcium chloride | -0.077 | 0.063 | -0.014 | -19 |
| Sodium proprionate | - | - | -0.017 | -23 |
| Urea | -0.018 | -0.01 | -0.028 | -38 |
| Sodium butyrate | - | - | -0.038 | -51 |
| Propylurea | - | - | -0.047 | -64 |
| Sodium thiocyanate | -0.077 | 0.007 | -0.07 | -96 |
| Potassium iodide | -0.083 | 0.01 | -0.073 | -100 |
| Sodium perchlorate | -0.097 | 0.021 | -0.076 | -104 |
| Sodium iodide | -0.087 | 0.01 | -0.077 | -105 |
| Guanidine hydrochloride | -0.061 | -0.027 | -0.088 | -120 |

Since thermodynamics is a description of the ultimate reality, the last column listing free energies of transfer shows those agents which are preferred in practicing the present invention, those agents with negative standard free energies. The Setschenow constants are helpful, however, in appreciating how the agent is useful. The "sum" column of interaction with peptide linkages in the polypeptide plus the interaction with the hydrophobic moieties is directly linked to the free energy column by the Nernst equation. It is the peptide interaction number and the hydrophobic or "methylene" number, which show how a dissociation agent works.

Urea, guanidine hydrochloride, or any other compound which has two negative parameters interact with the entire macromolecule to disaggregate the quaternary structure and perhaps unfold the secondary structure and are especially preferred for this invention. Sodium perchlorate, potassium iodide, and the like interact so strongly with peptide bonds that their lack of interaction with hydrophobic linkages of the protein does not appreciably inhibit dissociation or unfolding of the entire protein. These agents nay be useful in practicing the present invention. Ethanol, dioxane and other organics strongly react with the hydrophobic areas, usually the core of proteins, but not enough to overcome the nonpolar nature of organic solvents. Data on ethanol diverges, however. Such agents have limited utility in practicing this invention. Agents which have two positive components for their Setschenow constant and hence a positive standard free energy of transfer do not appear on Table I. Water-soluble amides with more than three carbon atoms are known also to be good dissociation agents but their Setschenow constants are not readily available and do not appear in Table I. They are also encompassed in the scope of the present invention.

Electrophoresis is the transport of both solute and solvent in an electric field. Ionophoresis is the transport of charged ions by coulombic attraction/repulsion in an electric field. Electroosmosis is the transport of solvent in an electric field.

Many workers in the prior art overemphasized ionophoresis and underestimated electroosmosis in their analysis of both the best modes for and problems associated with transdermal delivery of drugs by electrolytic means. In fact, the essence of transdermal, electric-powered delivery of drugs is that control and maximization is central regardless of whether the drug is transported by coulombic attraction/repulsion or electroosmotic solvent streaming. In the present invention, unlike the prior art, Faraday's law is irrelevant. In many situations, especially in the transdermal delivery of proteins such as insulin, more drug is carried by electroosmosis than ionophoresis, so that the amount of charge or degree of ionization of the protein is not important. Before the present invention this fact was not appreciated. Prior workers attempted to improve ionophoresis by increasing charge density on the protein by oxidation or hydrolysis. For this invention the value of charge density on the drug does not control the dosage.

Electronic conduction is the movement of electrons in an electric field. Electrolytic conduction is the movement of ions in an electric field. Prior to the present invention, many workers failed to communicate

6

their results well or to explain their ideas well because of confusion regarding the flow of electrons and the flow of ions. In the applicator of the present invention, current flow in the electrodes is electronic and current flow in the reservoir and through the skin is electrolytic, but it is possible to have some electronic flow along the chain of a protein in an electric field in water or aqueous media.

The values of the electrical variables in the practice of the current invention in vivo are those pertaining to electroosmosis not ionophoresis. The current density may be from about 0.5 $\mu A/cm^2$ to about 1 $mA/cm^2$, preferably about 0.5 microampere/$cm^2$ to about 10 microampere/$cm^2$ rather than the 1 milliamperes/$cm^2$ to 5 milliamperes/$cm^2$ values associated with ionophoresis. The voltage impressed for operating the applicator of the present invention ranges from about 1 to about 40 volts rather than the 50 to 100 or more volts advisable for ionophoresis. Likewise the migratory flow of water in an electrolytic field are the much higher values of about 0.001 $ml/cm^2/hr$ to about 0.005 $ml/cm^2/hr$ constant of electroosmosis not the typical adventitious values for ionophoresis, following Faraday's law which impels only ions.

It is highly preferred that the current density employed in the present invention be low enough to prevent any irritation, reddening, inflammation, or erythema in the skin of the patient.

The chemical composition of the present invention comprises three components: protein drug, cosolvent with negative Setschenow constant, and water. One can also consider the "cosolvent" as a cosolute. In addition, the composition may contain salts for physiological balance, buffering agents, disinfectants, antibiotics, preservatives, or other additives.

It is sometimes useful to add chelating agents to the chemical composition of this invention in order to demetallize a protein. Demetallization changes the quarternary structure of protein by removing metallic cations, which bind together polypeptide chains. Some of the metal ions which are integral parts of protein structure are magnesium, zinc, copper, chromium, cobalt, nickel, iron, and manganese. Many conventional chelating agents may be employed such as the salts of ethylenediaminetetraacetic acid (EDTA). Other conventional chelating agents may also be used.

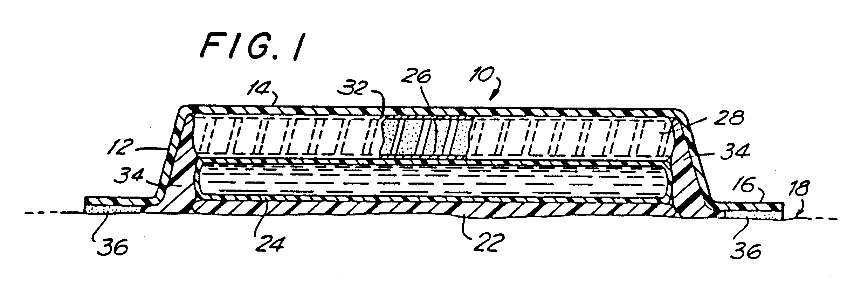

Figure 1 shows generally drug applicator 10 comprising outer cover 12 having a raised portion 14 and an outer-edge lip 16 in contact with the skin 18 of the patient. The layered structure of the drug applicator 10 can be any convenient and effective size or shape such as rectangle, oval, circle, or splayed shape to fit crevices at interfaces of body parts. The size of the applicator may range from about 10 $cm^2$ to about 200 $cm^2$ depending on its use and the species, age, and size of the patient.

Applicator 10 often has generally a structure of horizontal layers. The layer shown in Figure 1 as that closest to the skin 18 is an optional semipermeable membrane 22 through which the drug diffuses for deposition on skin 18. Optional membrane 22 may be constructed of semipermeable, cellulose acetate, poly(vinyl chloride, or regenerated cellulose.

Above optional semipermeable membrane 22 is a reservoir, region, or pouch 24 for holding the supply of the protein to be electrolytically delivered and a reservoir for the other electrode. Preferably reservoir 24 defines a closed space and is flexible. Typical materials used in farming pouch 24 are rayon floc, polyurethane sponge, and hydrophilic adhesive in latex form. This reservoir may also consist of a hydrophilic gel. For containing the protein solution or suspension of the present invention, reservoir 24 may range from about 0.01 ml to about 15 ml in volume, preferably about 0.15 ml to about 0.9 ml for about a week's continual administration of a protein drug in amounts ranging from about 500 nanograms to 1 mg per day, depending on the size, species, and age of the patient. The gel, pouch, or walls of the reservoir 24 must be microporous enough for transfer of the solvent, solution, or suspension of the protein by the electric field, but not so porous to allow leakage of the suspension or solution of the protein drug. The choice of whether or not to employ optional semipermeable membrane 22 is interrelated with the choice of design and material of reservoir 24, because their functions may overlap.

The next higher layer above reservoir 24 as shown in Figure 1 comprises extended contact 26 which is preferably the lower face of battery 28. Contact 26 preferably is flexible enough to conform to the surface of the skin and also is electronically conducive. Preferred materials for contact 26 are electric-conducting polymers, carbonized plastic films, or plastic surfaces loaded with or painted with highly conducive powdered or solid carbon or graphite.

Battery 28 comprising the next layer may be made up of a group of cells internally connected in series to obtain the desired voltage necessary to obtain the electrophoretic action with the particular protein. Orientation of battery 28 depends on the direction of endosmotic flow which is usually from the anode. With regard to battery 28, it should be noted that any conventional miniaturized battery cells now generally available can be employed, arrange and connected in series to obtain the desired operating voltage. In addition, the technology now exists for batteries made of thin, flexible sheets of an electrically conductive polymer with high surface area relative to its thickness to provide adequate current densities. One such so-called plastic battery is described in "Batteries Today", Autumn 1981, pages 10, 11, and 24. When such a battery is employed, sheets may be layered to place the cells in series, and an effective compromise between number of sheets and surface areas of sheets is achieved by layering them diagonally, as shown somewhat schematically in Fig. 1. Of course, battery selection also depends on such factors as the degree of conformability desired, voltage and current densities required for a specific application, and time of discharge.

In Figure 1, above battery 28 is electrical contact 32, which preferably is similar in design and material to electrical contact 26 and forms the opposite side of the battery.

Cover 12 encloses all the previously listed layers of drug applicator 10 and is made of flexible, conductive material such as a plastic polymer impregnated with carbon, electrically conductive itself, or carbonized on its surface. Insulating material 34 fills the space between the side walls of raised portion 14 and the various aqueous layers containing electrolyte. Suitable insulating materials are polyester, silicones, and any other drug-compatible plastics. Alternatively, a totally insulating cover may envelope all of the working components previously named..

In order for drug applicator 10 to make good contact with and stick to the patient's skin 18 electrically-conductive adhesive 36 is applied under the edge of lip 16. Suitable conducting adhesive materials are those filled with powdered conductors such as carbon or graphite.

It will be seen that the arrangement described above forms a complete electric circuit from one side of battery 28, cover 12, adhesive material 36, skin 18, microporous membrane 22, liquid reservoir 24, and back to battery 28. Also the reservoir may be divided into separate anode and cathode compartments with an insulator between and the batery in a separate compartment.

The electrical operation of the drug applicator may be carried out in many modes, including that of uniform direct current. The impressed voltage from the power source may be pulsed with a wide variety of pulse width and frequency. A saw-tooth voltage or other types of reversing, sinusoidal, or alternating voltage sources are also within the disclosure of this invention.

The types of batteries and their orientation are disclosed inter alia in U.S. 4,557,723 and 4,640,689. The types of circuits which may be employed are also disclosed in various of the above-cited related applications.

The preferred proteins to be administered by the composition of the present invention are compounds, such as insulin, protamine, glucagon, calcitonin, proteinaceous adrenal hormones, and the like. Other proteins such as albumin may be administered transdermally by an electrolytic device. Proteins related to blood fractions such as globulins, tetanus, rabies and other proteins or antibodies may also be employed, as may be enzymes or other proteinaceous entities.

Quite arbitrarily this disclosure terms any polypeptides with more than about 20 alphaaminoacids, as a "protein". Thus glucagon (29 units), calcitonin (32 units), and corticortropin (39 units) are here termed "protein", as is disassociated insulin (51 units). Polypeptides from about 3 to about 20 alphaamino acid units are termed "polypeptides" and are not preferred in the practice of the present invention.

Having described the inventive composition of protein dissociating agent with negative Setschenow constants, and aqueous electrolyte and having described an embodiment of the electrolytic drug applicator for tramsdermal delivery of proteins, we now illustrate the invention in the following Examples, These Examples, however, are intended only to illustrate not limit the scope of the instant invention, which may be carried out by other means and still be covered by the teachings of this disclosure.

EXAMPLE 1

This Example illustrates the preparation of small electrolytic transdermal devices with side-by-side reservoirs and electrodes. Another possible design is that of a "matted-photograph" with the drug reservoir anode surrounded by an insulated frame-shaped cathode, as shown in Figure 1.

The side-by-side reservoirs and electrodes have a rayon gauze next to the skin (Johnson & Johnson Co., New Brunswick, New Jersey). Two matted rayon pads 5 cm x 8 cm x 0.5 cm are topped by U-shaped polyester film 0.1 mm thick coated with 0.02 mm layer of conducting graphite paint (Bertek Corp., St. Albans, Vermont) surrounding a central insulator of 0.2 mm Mylar polyester film (duPont Co., Wilmington, Delaware). The top surface of the U-shaped graphitized polyester film is connected to a 9V battery (El Power Corp., Santa Anna, California). The periphery of the felted reservoir pads and electrodes plus an insulating band in the gauze base between then is RTV silicone resin (Dow Corning Co., Midland, Michigan). Surrounding the top and sides of the device is surgical adhesive tape (Hy-Tape Surgical Hosiery Corp., New York, New York). Each of the reservoirs can hold 6 ml of aqueous fluid.

Example 2

This Example (86702) illustrates the transdermal delivery of insulin by the composition of the present invention to rabbits employing urea.

Eight healthy albino rabbits were equilibrated for 24 days under standard clinical conditions. Their backs were clipped the day before, washed with castille soap on the day of the test, and a 7.5 cm x 10 cm electrolytic patch containing two 6 ml reservoirs applied, as prepared in Example 1. The negative reservoir contained 6 ml with 500 IU of regular human insulin (Lilly, Humilin R) and 1% (0.16 M) urea. The positive reservoir contained 5 ml of 0.9% saline. The patches were held next to the clipped skin with elastic tape, and the rabbits restrained for the 10 hours of the test.

The blood was collected from the medial artery of each rabbit's ear at 0,4,5,6,7,8,9, and 10 hours of the test. Blood glucose was determined with an Accu-Check II blood glucose monitor (Boehringer Manheim Co., Ridgefield, Conn.). Radioimmuno assays (RIA) were performed with a Pharmacia Corp. (Piscataway, New Jersey) Insulin 100 RIA kit.

Radioactivity values were standardized and counted on a Tracor Corp. (Austin, Texas) gamma counter.

Patch Model Y provided twice the current density as patch model X.

The insulin data for all eight rabbits are shown in Table 2A for this Example. One rabbit with patch Y model died at the eight-hour point from insulin overdose. Inspection of Table 2A shows that transdermal delivery was effective in five of the eight rabbits and marginal in one for this period of time.

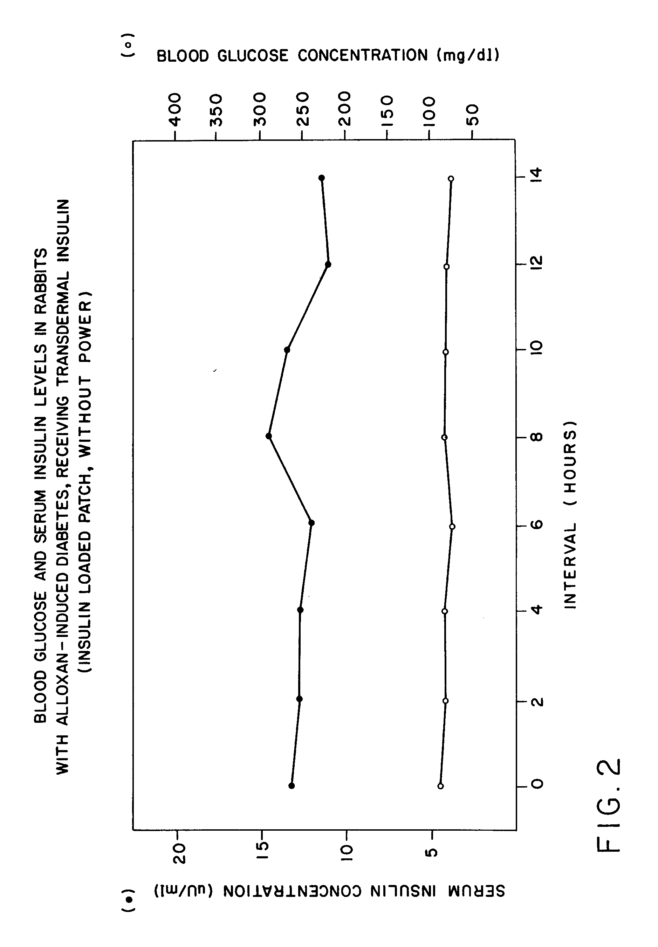

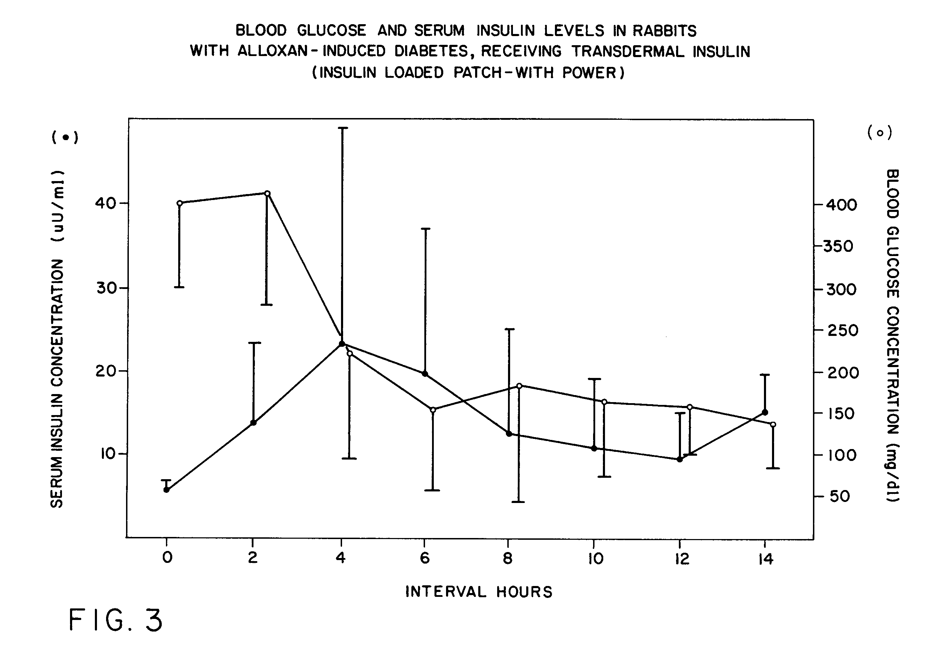

Table 2B shows blood glucose values for all eight rabbits at the various test points. Inspection of Table 2B shows that glucose levels were lowered in varying degree in seven of the eight rabbits. See fig. 2 and 3.

TABLE 2A

| SUMMARY OF INSULIN CONCENTRATION IN SERUM FOLLOWING TRANSDERMAL DELIVERY OF INSULIN CONTAINING 1 MG UREA PER ML | | | | | | | | | |
|---|---|---|---|---|---|---|---|---|---|
| Rabbit No. | Patch Model | Insulin Concentration in Serum (uU/ml Interval (Hours) | | | | | | | |
| | | 0 | 4 | 5 | 6 | 7 | 8 | 9 | 10 |
| 1281 | X | 6.9 | 57.1 | 58.1 | 35.4 | 37.3 | 30.0 | 21.0 | 29.3 |
| 1282 | X | 3.3 | 4.2 | 3.7 | 4.2 | 4.0 | 4.0 | 3.7 | 4.2 |
| 1284 | X | 4.4 | 4.4 | 4.1 | 4.4 | 4.0 | 3.8 | 3.8 | 4.2 |
| 1285 | X | 3.7 | 12.3 | 11.6 | 12.8 | 11.6 | 8.8 | 11.2 | 12.6 |
| 1286 | Y | 3.8 | 3.9 | 3.9 | 3.5 | 3.9 | 3.8 | 3.7 | 5.5 |
| 1287 | Y | 3.6 | 4.5 | 7.6 | 5.8 | 8.5 | 9.3 | 24.0 | 175 |
| 1288 | Y | 3.5 | 9.9 | 13.7 | 10.4 | 13.4 | 11.4 | 11.7 | 28.7 |
| 1289 | Y | 6.9 | 187.0 | 197 | 217 | 211 | 198[a] | | |

a Animal died at 8 hours.

TABLE 2B

| SUMMARY OF BLOOD GLUCOSE LEVELS FOLLOWING TRANSDERMAL DELIVERY OF INSULIN CONTAINING 1 MG UREA PER ML | | | | | | | | | | |
|---|---|---|---|---|---|---|---|---|---|---|
| Rabbit No. | Patch Model | Blood Glucose Concentration (mg/dl) Interval (Hours) | | | | | | | | |
| | | 0 | 4 | 5 | 6 | 7 | 8 | 9 | 10 |
| 1281 | X | 131 | 79 | 65 | 64 | 58 | 47 | 39 | 32 |
| 1282 | X | 133 | 100 | 131 | 110 | 120 | 114 | 113 | 97 |
| 1284 | X | 134 | 124 | 132 | 121 | 112 | 113 | 120 | 117 |
| 1285 | X | 107 | 103 | 101 | 92 | 94 | 87 | 82 | 80 |
| 1286 | Y | 115 | 121 | 135 | 128 | 137 | 127 | 129 | 122 |
| 1287 | Y | 135 | 136 | 140 | 121 | 101 | 88 | 82 | 82 |
| 1288 | Y | 138 | 115 | 104 | 94 | 90 | 75 | 79 | 67 |
| 1289 | Y | 119 | 38 39[a] | 22 | 29 | 57 | 35[b] | - | - |

a Sample repeated to confirm original assey
b Animal died at 8 hour interval

COMPARATIVE EXAMPLE 1

This Comparative Example illustrates the necessity of having a compound with negative Setschenow constants in the composition of the drug reservoir in order to achieve appreciable delivery or a protein such as insulin to the bloodstream of the patient. Without a dissociating agent only very small amounts of insulin are delivered by electroosmosis.

Employing patches prepared by the procedure of Example 1 and insulin tests as in Example 2, four albino rabbits were treated with a patch whose anode reservoir held 5 ml containing 500 IU of Humilin R (Lilly Co., Indianapolis, Ind.) but no dissociating agent and whose cathode compartment contained 0.9% saline.

Table CE shows the insulin values in the rabbit blood samples at the hour points indicated. Because the insulin transport was so low without the dissociating agent of the present invention, blood glucose data were not obtained.

Disregarding the two aberrant values, inspection of Table CE shows that only two rabbits (Nos. 367 and 547) received a measurable dosage or insulin.

TABLE CE

| Rabbit No. | Insulin Values (uU/ml) at Intervals (Hours) | | | | | | |
|---|---|---|---|---|---|---|---|
| | 0 | 1 | 2 | 3 | 4 | 5 | 6 |
| 364 | h | 5.9 | 3.6 | 3.6 | 4.1 | 4.7 | 4.6 |
| 365 | 4.1 | 4.7 | 3.9 | h | - | 3.4 | 10.8 h |
| 367 | 13.5 h | 10.0 | 11.3 | 28.1 | 33.3 h | 25.4 | (104)sic |
| 385 | 14.6 h | 3.4 | 5.3 | 7.4 | 6.3 | 4.9 | (231)sic |
| 545 | 10.7 | 8.0 | 5.1 | 7.7 | 8.3 | 6.9 | 14.2 |
| 547 | 5.4 | 5.2 | 9.9 | 9.9 | 12.8 | 18.6 h | 17.5 |
| 549 | 12.3 | 10.5 | 7.4 | 7.0 | 8.2 | 10.8 | 12.0 |
| 551 | 3.0 | 3.7 h | 3.2 | 10.1 | 4.2 | 4.7 | 5.4 |
| h shows samples with marked hemolysis | | | | | | | |

MODEL EXAMPLES

Model Example 1

This Example illustrates the use of a protein composition of the present invention in an electrolytic transdermal applicator to obviate the use of a series of injections and to introduce a protein drug to the bloodstream of the patient gradually without the trauma of one or more bolus injections.

Twelve electrolytic patches as in Example 1 employing a positive drug reservoir of 6 ml contain 2 mg activity protamine sulfate (Lilly, Injection USP) per ml, 1.5 % sodium iodide, and the 0.9% sodium chloride found in commercial USP protamine. The negative reservoir contains 0.9% saline.

The standard blood clotting time of each of 12 albino rabbits, prepared as in Example 2 is determined. At zero time the 12 rabbits are fired with the electrolytic transdermal patches employed in Example 2. The six control patches have no batteries. At the three-hour point all 12 rabbits are injected intravenously with 500 units of Heparin Sodium Injection USP (Upjohn Co., Kalamazoo, Mich.). At the three-hour 20-minute point the coagulation time of a 1 ml sample of blood taken from all 12 rabbits is measured.

It is found that the rabbits fitted with the operating protamine protein electrolytic transdermal patch of this invention have a clotting time considerably shorter than those control rabbits with the protamine sulfate transdermal patch inoperative. One notes that rapid injection of protamine may cause dyspnea, flushing, bradycardia, and hypotension in humans.

Model Example 2

This Example illustrates the' use of a larger-sized electrolytic patch for administering calcitonin, a 32-unit polypeptide to human adults to lower the calcium level in their blood and inhibit bone resorption (Paget's disease). As a beneficial side effect slow delivery of calcitonin improves impaired auditory nerves, lowers high cardiac output, and treats postmenopausal osteoporosis.

In the same manner as in Example 1, electrolytic patches are prepared based on 12 cm by 8 cm by 0.6 cm felted rayon pads. Three 3-volt, nickel-cadmium, wafer batteries are used in series on the top. The entire side periphery and cover is made from 0.15mm PVC film. No semipermeable ultrafiltration membrane is employed between the bottom of the two side-by-side reservoirs and the skin.

The drug reservoir compartment comprises 12 ml Calcimar synthetic salmon-calcitonin (USV, Tarrytown, New York) containing per ml 200 IU calcitonin-salmon, 5 mg. phenol biocide, and trace amounts of sodium chloride, sodium acetate, acetic acid, and sodium hydroxide for buffering and tonicity all in 0.2 M sodium perchlorate, which has a negative Setschenow constant. The current density is 5 $\mu$A/cm$^2$, which delivers 50 IU per hour.

Monitoring four adult male subjects over a 20-day period shows a lowering of calcium in the subjects averaging 12%, a distinct advantage over repeated injections which lowers blood calcium level only 9% in general.

Model Example 3

This Example illustrates the use of the composition of the present invention to deliver glucagon electolytically and transdermally in patients without the necessity of repeated injections in order to control hyperglycemia and avoid insulinoma and/or pheochromocytoma with decreased probability of the release of catcehol amines. Glucagon is a protein with 29 peptide units and comprises aminoacids 33 through 61 of glicentin. In vivo glucagon is synthesized multistranded with a molecular weight about 18,000. The commercial extract from beef and pork pancreas is single-stranded with a molecular weight of 3483.

As in Model Example 1, six rabbits are prepared and fitted with the electrolytic transdermal patches, as in Example 2. The "return" reservoir contains 0.9% saline solution. The drug reservoir contains 100 units of Lilly Co. No. 668 Glucagon for Injection USP in 0.2 m propylurea, which has a negative Setschenow constant. A current density of 3 $\mu$A/cm$^2$ is loin enough to allow a controlled rise in blood sugar values. Comparison of zero time blood sugar values with 8-, 16-, 24- etc. hour values for four days shows an increase in blood sugar value averaging about 12%.

Model Example 4

This Model Example illustrates the delivery of corticotropin at continuous low levels transdermally by the composition of the present invention. Human, ovine, porcine, and bovine adrenocorticotropic hormone (ACTH) are all 39-unit polypeptides differing slightly in composition at aminoacids in the 25, 31, and 33 positions.

By the same procedures employed in Model Example 3 ACTHAR-40 (Armour Co., Tarrytown, New York) is introduced to eight rabbits whose levels of cortisol, corticosterone, and aldosterone in the blood are monitored every six hours for eight days. A current density of 5 $\mu$A/cm$^2$ introduces the drug transdermaly at the rate of 1.5 USP units/hour. The drug reservoir contains 400 units in 0.25 M potassium iodide, which has a negative Setschenow constant.

Comparison of the cortisol, corticosterone, and aldosterone levels in these rabbits shows an average increase of about 15%, when the ACTHAR is being administered, compared to the values before this eight-day test of the electrolytic transdermal device.

**Claims**

1. Use of a mixture comprising a protein, water and a dissociating agent with a negative Setschenow constant for the manufacture of a composition for use in transdermal transport to the blood stream of a patient by electronic means at a current density between 0,5 $\mu$A/cm$^2$ and 10 mA/cm$^2$, impressed voltage between 1 and 40 V, and a migrating water volume value between 0,001 ml/cm$^2$/h and 0,01 ml/cm$^2$/h.

2. Use according to claim 1, wherein the current density is between 5 $\mu$A/cm$^2$ and 1 mA/cm$^2$, the voltage is between 3 and 10 V, and the migrating water volume is between 0,002 and 0,005 ml.

3. Use according to claim 1 or 2, wherein the dissociating agent is selected from the group consisting of urea, alkylderivatives of urea, guanidine salt, butanol, 2-butanol, water-soluble amides with more than three carbon atoms, any water-soluble salt with a negative Setschenow constant, and mixtures thereof, especially wherein the dissociating agent is selected from the group consisting of urea, propylurea, and guanidine hydrochloride.

4. Use according to one of the preceding claims, wherein the protein is selected from the group consisting of glucagon, protamines, adrenal cortex proteinaceous hormones, calcitonin, albumins, globulins, insulins, and mixtures thereof, especially wherein the protein is an insulin.

5. Use according to one of the preceding claims, wherein the protein is any polypeptide having more than 20 polypeptide units.

6. Use according to one of the preceding claims, wherein the mixture further comprises a chelating agent.

7. Use according to one of the preceding claims, wherein the mixture further comprises a buffering agent.

8. Use according to one of the preceding claims, wherein the mixture further comprises a biocide for preserving the mixture.

9. A transdermal patch for the application of a composition manufactured by use of a mixture comprising a protein, water and a dissociating agent with a negative Setschenow constant, according to any of the preceding claims, comprising a hydrophilic reservoir containing the composition, especially wherein the reservoir holds from 0,01 ml to 15 ml of the composition.

10. A transdermal patch according to claim 9, further comprising an electric battery and two extended contacts.

11. A transdermal patch according to claims 9 or 10, further comprising a semipermeable membrane on the skin side of the reservoir.

12. A transdermal patch for delivering at least one protein drug to the blood stream of the patient comprising in combination:
    a) electrolytic means comprising a battery, an anode, a cathode, a protein drug reservoir, and barrier means between the electrodes, and
    b) a pharmaceutical composition for transdermal transport comprising a protein, water, and a dissociating agent with a negative Setschenow constant,
    whereby the patch operates at a current density between about 0,5 microA/cm$^2$ and about 10

milliA/cm$^2$.

## Revendications

1. Utilisation d'un mélange comprenant une protéine, de l'eau et un agent de dissociation ayant une constante de Setschenow négative, dans la fabrication d'une composition utilisable pour le transport transdermique dans le circuit sanguin d'un patient par des moyens électroniques, à une densité de courant comprise entre 0,5 microA/cm$^2$ et 10 milliA/cm$^2$, une tension appliquée entre 1 et 40 in et un volume d'eau migrante compris entre 0,001 ml/cm$^2$/h et 0,01 ml/cm$^2$/h.

2. Utilisation selon la revendication 1, dans laquelle la densité de courant est comprise entre 5 microA/cm$^2$ et 1 milliA/cm$^2$, le voltage entre 3 et 10 V et le volume d'eau migrante entre 0,002 et 0,005 ml.

3. Utilisation selon la revendication 1 ou 2, dans laquelle l'agent de dissociation est choisi dans le groupe consistant en l'urée, les dérivés alkylés de l'urée, les sels de guanidine, le butanol, le 2-butanol, les amides solubles dans l'eau ayant plus de 3 atomes de carbone, n'importe quel sel soluble dans l'eau ayant une constante de Setschenow négative, et leurs mélanges, plus particulièrement dans lesquels l'agent de dissociation est choisi dans le groupe consistant en l'urée, la propylurée, et le chlorhydrate de guanidine.

4. Utilisation selon l'une des revendications précédentes, dans laquelle la protéine est choisie dans le groupe consistant en le glucagon, les protamines, les hormones protéiques du cortex adrénal, la calcitonine, les albumines, les globulines, les insulines, et leurs mélanges, plus particulièrement dans laquelle la protéine est une insuline.

5. Utilisation selon l'une des revendications précédentes, dans laquelle la protéine est n'importe quel polypeptide ayant plus de 20 unités polypeptidiques.

6. Utilisation selon l'une des revendications précédentes, dans laquelle le mélange comprend en outre un agent de chélation.

7. Utilisation selon l'une des revendications précédentes, dans laquelle le mélange comprend en outre un agent tampon.

8. Utilisation selon l'une des revendications précédentes, dans laquelle le mélange comprend en outre un biocide de conservation pour le mélange.

9. Timbre transdermique pour appliquer une composition fabriquée en utilisant un mélange comprenant une protéine, de l'eau et un agent de dissociation avec une constante de Setschenow négative, selon l'une des revendications précédentes, comprenant un réservoir hydrophile contenant la composition, plus particulièrement dans laquelle le réservoir contient de 0,01 ml à 15 ml de la composition.

10. Timbre transdermique selon la revendication 9, comprenant en outre une pile électrique et deux contacts en prolongement.

11. Timbre transdermique selon la revendication 9 ou 10, comprenant en outre une membrane semi-perméable du côté du réservoir en contact avec la peau.

12. Timbre transdermique pour la délivrance d'au moins un produit pharmaceutique protéique dans le circuit sanguin d'un patient, comprenant en combinaison :
    a) des moyens électrolytiques comprenant une pile, une anode, une cathode, un réservoir pour le produit pharmaceutique protéique, et des moyens de barrière entre l'électrode et,
    b) une composition pharmaceutique pour le transport transdermique comprenant une protéine, de l'eau et un agent de dissociation ayant une constante de Setschenow négative,
    dans laquelle le timbre fonctionne sous une densité de courant comprise entre 0,5 microA/cm$^2$ environ et 10 milliA/cm$^2$ environ.

## Patentansprüche

**1.** Verwendung einer Mischung aus Protein, Wasser und einem dissoziierenden Agens mit einer negativen Setschenow-Konstante für die Herstellung eines Präparats zum Verwenden beim transdermalen Transport zu dem Blutstrom eines Patienten durch elektronische Mittel bei einer Stromdichte zwischen 0,5 $\mu A/cm^2$ und 10 $mA/cm^2$, einer eingeprägten Spannung zwischen 1 und 40 V und einem Volumenswert für migrierendes Wasser zwischen 0,001 $ml/cm^2/h$ und 0,01 $ml/cm^2/h$.

**2.** Verwendung nach Anspruch 1, bei welcher die Stromdichte zwischen 5 $\mu A/cm^2$ und 1 $mA/cm^2$, die Spannung zwischen 3 und 10 V und das Volumen für migrierendes Wasser zwischen 0,002 und 0,005 ml liegen.

**3.** Verwendung nach Anspruch 1 oder Anspruch 2, bei welcher das dissoziierende Agens aus der Gruppe ausgewählt ist, die Harnstoff, Alkylderivate von Harnstoff, Guanidinsalz, Butanol, 2-Butanol, wasserlösliche Amide mit mehr als drei Kohlenstoffatomen, irgendein wasserlösliches Salz mit einer negativen Setschenow-Konstante und Mischungen derselben enthält und insbesondere bei welcher das dissoziierende Agens aus der Gruppe ausgewählt ist, die Harnstoff, Propylharnstoff und Guanidinhydrochlorid enthält.

**4.** Verwendung nach einem der vorgehenden Ansprüche, bei welcher das Protein aus der Gruppe ausgewählt ist, die Glucagon, Protamine, eiweißartige Hormone der Nebennierenrinde, Kalzitonine, Albumine, Globuline, Insuline und Mischungen derselben enthält und insbesondere bei welcher das Protein ein Insulin ist.

**5.** Verwendung nach einem der vorgehenden Ansprüche, bei welcher das Protein irgendein Polypeptid mit mehr als 20 Polypeptideinheiten ist.

**6.** Verwendung nach einem der vorgehenden Ansprüche, bei welcher die Mischung ferner ein chelatbildendes Agens enthält.

**7.** Verwendung nach einem der vorgehenden Ansprüche, bei welcher die Mischung ferner ein Pufferungsagens enthält.

**8.** Verwendung nach einem der vorgehenden Ansprüche, bei welcher die Mischung ferner ein Biozid enthält, um die Mischung zu konservieren.

**9.** Transdermales Pflaster für die Applikation eines Präparats, das durch Verwenden einer Mischung, die ein Protein, Wasser und ein dissoziierendes Agens mit einer negativen Setschenow-Konstante enthält, gemäß einem der vorangehenden Ansprüche hergestellt ist, das einen hydrophilen Behälter aufweist, welcher das Präparat enthält und insbesondere bei welchem der Behälter von 0,01 ml bis 15 ml des Präparats enthält.

**10.** Transdermales Pflaster nach Anspruch 9, das ferner eine elektrische Batterie und zwei ausgedehnte Kontakte aufweist.

**11.** Transdermales Pflaster nach Anspruch 9 oder 10, das ferner eine semipermeable Membran auf der Hautseite des Behälters aufweist.

**12.** Transdermales Pflaster zum Liefern zumindest eines Proteinmedikaments zu dem Blutstrom des Patienten, das in Kombination folgendes aufweist:
a. elektrolytische Mittel, die eine Batterie, eine Anode, eine Kathode, einen Behälter für Proteinmedikamente und Barriere-Mittel zwischen den Elektroden aufweisen und
b. ein pharmazeutisches Präparat für transdermalen Transport, das ein Protein, Wasser und ein dissoziierendes Agens mit einer negativen Setschenow-Konstante enthält,
wodurch das Pflaster bei einer Stromdichte zwischen etwa 0,5 $mikroA/cm^2$ und etwa 10 $milliA/cm^2$ arbeitet.

FIG. 1

EP 0 278 473 B1

BLOOD GLUCOSE AND SERUM INSULIN LEVELS IN RABBITS
WITH ALLOXAN-INDUCED DIABETES, RECEIVING TRANSDERMAL INSULIN
(INSULIN LOADED PATCH, WITHOUT POWER)

FIG. 2

BLOOD GLUCOSE AND SERUM INSULIN LEVELS IN RABBITS
WITH ALLOXAN - INDUCED DIABETES, RECEIVING TRANSDERMAL INSULIN
(INSULIN LOADED PATCH - WITH POWER)

FIG. 3